# EUROPÄISCHE PATENTSCHRIFT

(11) **EP 2 150 352 B1**
(45) Veröffentlichungstag und Bekanntmachung des Hinweises auf die Patenterteilung: **18.05.2011**
(21) Anmeldenummer: 08760506.9
(22) Anmeldetag: 04.06.2008
(51) Int. Cl.: B04B 5/04

(54) **EINSATZ UND ZENTRIFUGE MIT EINSATZ**
INSERT AND CENTRIFUGE COMPRISING AN INSERT
PIECE RAPPORTEE ET CENTRIFUGEUSE A PIECE RAPPORTEE

(30) Priorität: 05.06.2007 DE 102007000308
(43) Veröffentlichungstag der Anmeldung: 10.02.2010
(73) Patentinhaber: Terumo Europe NV, 3001 Leuven (BE); Andreas Hettich GmbH&Co. KG, 78532 Tuttlingen (DE)
(72) Erfinder: EBERLE, Klaus-Günter, 78532 Tuttlingen (DE); BISET, Roland, B-3001 Leuven (BE)
(74) Vertreter: TBK
(86) Internationale Anmeldenummer: PCT/EP2008/056926
(87) Internationale Veröffentlichungsnummer: WO 2008/148811

(56) Entgegenhaltungen:
- EP-B- 1 351 772
- US-A1- 2002 085 957

## Beschreibung

### Technisches Gebiet

Die Erfindung betrifft einen Einsatz, der insbesondere zur Verwendung in einer Zentrifuge vorgesehen ist, um Blut in seine Bestandteile aufzutrennen, und eine Zentrifuge mit einem solchen Einsatz.

### Stand der Technik

In der Transfusionsmedizin hat sich seit dem Beginn der neunziger Jahre die so genannte Blutkomponententherapie durchgesetzt. Dies bedeutet, dass einem Patienten anstelle einer Vollblutkonserve lediglich diejenigen Blutbestandteile verabreicht werden, die der einzelne Patient benötigt. Durch diese getrennte Verabreichung der einzelnen Blutbestandteile ist es möglich, mit einer einzigen Blutkonserve durchschnittlich 1,8 Patienten optimal zu helfen.

Die wesentlichen Blutbestandteile sind
die roten Blutkörperchen im so genannten Erythrozytenkonzentrat, die zur Aufrechterhaltung der Sauerstoffversorgung nach schweren Blutverlusten transfundiert werden,
die Blutplättchen im Thrombozytenkonzentrat, die bei Gerinnungsstörungen (Bluterkrankheit) verabreicht werden, und
das Blutplasma, das bei Gerinnungsstörungen und Volumendefiziten verabreicht wird. Abgesehen davon ist Blutplasma ein wesentlicher Grundbestandteil zur Herstellung vieler Medikamente.

Das Auftrennen der einzelnen Blutbestandteile, das als Zellgewinnung bezeichnet wird, erfolgt bekannt durch ein Behandeln des Bluts in einer Zentrifuge. Durch das Zentrifugieren werden die einzelnen Blutbestandteile voneinander getrennt und können dann einzeln in entsprechende Behältnisse abgefüllt und verwendet werden.

Beispielsweise ist aus der EP 1 351 772 B1 eine derartige Zentrifuge bekannt. Gemäß diesem Stand der Technik sind in einem Rotor einer Zentrifuge eine Vielzahl von Einsätzen um eine Nabe angeordnet. Die Einsätze sind dabei fest in dem Rotor gehalten, sodaß die Blutbeutel stehend zentrifugiert werden. Die Einsätze weisen in Ihrem Inneren Aufnahmen für einen Vollblut enthaltenden Blutbeutel und für Produktbeutel auf, in denen das Plasma bzw. das Erythrozytenkonzentrat gesammelt wird. Um zu verhindern, dass es nach erfolgter Trennung der einzelnen Bestandteile zu einem Nachfließen und abermaligen Vermischen der Produkte kommt, sind in dem Einsatz verschiedene Klemmeinrichtungen vorgesehen, mit denen die einzelnen Schläuche abgeklemmt werden können. Zum Entnehmen der Beutel aus dem Einsatz nach erfolgter Trennung, müssen die einzelne Anschlusschläuche der Beutel durch geeignete Maßnahmen verschlossen werden. Erst danach können die Klemmen des Einsatzes geöffnet, die Beutel entnommen und der Einsatz zur Aufnahme eines neuen Beutelsatzes vorbereitet werden.

Nach der Trennung und dem Abfüllen des Plasmas bzw. der roten Blutkörperchen verbleibt ein als "buffy coat" bezeichnetes Gemisch in den Blutbeuteln. Dieses "buffy coat" besteht im Wesentlichen aus Blutplättchen sowie weißen und roten Blutkörperchen. Zur Gewinnung der Blutplättchen aus diesem "buffy coat" wird dieses mit einer Additivlösung verdünnt und dieses verdünnte "buffy coat" durch Zentrifugieren abermals in seine Bestandteile aufgetrennt.

Aus der WO 03/089027 ist ein System und Verfahren zu diesem Zweck bekannt. Diese Druckschrift offenbart eine Zentrifuge, in deren einzelner Kammer ein ringförmiger Beutel mit einem Gemisch aus "buffy coat" und Additivlösung eingelegt werden. Die Blutbestandteile werden dann durch einen Zentrifugiervorgang aufgetrennt und die getrennten Bestandteile durch eine Schlauchleitung über einen in dem Bereich der Nabe angeordneten Filter zu einem ebenfalls in dem Bereich der Nabe angeordneten Sammelbehälter geleitet.

### Darstellung der Erfindung

### Technische Aufgabe

Es ist die Aufgabe der Erfindung, eine verbesserten Einsatz und eine Zentrifuge mit dem Einsatz zu schaffen, die eine wirtschaftlichere Zellgewinnung ermöglichen.

### Technische Lösung

Die Aufgabe der Erfindung wird durch einen Einsatz nach Anspruch 1 durch eine Zentrifuge nach Anspruch 10 bzw. durch ein Verfahren nach Anspruch 17 gelöst. Vorteilhafte Ausführungsformen der Erfindung werden gemäß der abhängigen Ansprüche gelöst.

Ein erfindungsgemäßer Einsatz zur Aufnahme von Blutbeuteln, die zum Einsatz in einer Zentrifuge zur Trennung von Blutbestandteilen vorgesehen ist, weist eine Zwischenwand und eine Abdeckung auf. Die Zwischenwand teilt einen radial innen liegenden Blutbeutelbereich von einem radial außen liegenden Produktbereich ab. In einer Einbauposition des Einsatzes liegt die Abdeckung über dem Blutbeutelbereich. Die Abdeckung ist an einem Punkt drehbar und an einem zweiten Punkt lösbar mit der Zwischenwand verbunden. Dadurch ist der Blutbeutelbereich durch ein seitliches Wegdrehen der Abdeckung frei zugänglich.

Die ermöglicht ein schnelles Austauschen des Blutbeutels in dem Blutbeutelbereich. Durch die drehbare Verbindung der Abdeckung mit der Zwischenwand können ein oder mehrere Anschlussschläuche des Blutbeutels einfach positioniert werden, während die Abdeckung geschlossen wird. Dadurch sind die Schläuche binnen kurzer Zeit optimal fixiert.

Vorteilhaft kann in einem oberen Bereich der Abdeckung eine Aussparung zur Aufnahme eines Schlauchs des Blutbeutels und/oder einer Schlauchklemme ausgebildet sein. Diese Aussparungen ermöglichen die Verwendung der ursprünglichen Blutbeutel, ohne dass weitere Maßnahmen zur Vorbereitung des Beutels oder Schlauchs erforderlich sind.

Es kann in der Abdeckung eine Betätigungseinrichtung vorgesehen sein, die zum Betätigen einer auf einem Schlauch des Blutbeutels vorhandenen Schlauchklemme vorgesehen ist. Da die bereits auf dem Schlauch befindlichen Klemmen durch die Betätigungseinrichtung geöffnet und geschlossen werden können, ist es nicht notwendig, mit dem Einsatz eigene Klemmeinrichtungen vorzusehen.

Zur einfachen Betätigung der Klemme kann die Betätigungseinrichtung an einer bei geschlossener Abdeckung der Zwischenwand gegenüberliegenden Seitenfläche aus der Abdeckung vorstehen.

In der Aussparung kann ein Sensor vorgesehen sein, der insbesondere als lichtempfindlicher Sensor ausgeführt sein kann. Dadurch ist es möglich, je nach der Farbe der durch den Schlauch geleiteten Blutflüssigkeit entsprechende Reaktionen herbeizuführen, wie z.B. die Klemme zu betätigen, um die Abfuhr der Blutflüssigkeit aus dem Beutel zu beenden.

An einer Unterseite der Abdeckung kann eine elektrische Verbindungseinrichtung zur elektrischen Verbindung mit einer Zentrifuge vorgesehen sein. Dadurch ist es möglich, Signale von einem in der Zentrifuge eingebauten Steuergerät zu dem Einsatz bzw. in der umgekehrten Richtung zu übertragen.

In dem Produktbereich des Einsatzes kann außerdem eine Aufnahme für einen Filter und/oder einen Produktbeutel vorgesehen sein. Entsprechend kann nach dem Öffnen der Abdeckung der teilweise oder vollständig entleerte Blutbeutel mit dem verbleibenden Inhalt zusammen mit dem Filter und dem Produktbeutel, der z.B. das Plasma oder das Erythrozytenkonzentrat enthält, aus dem Einsatz entnommen werden.

Vorteilhaft kann die Aufnahme eine radial außerhalb der Zwischenwand liegende Außenwand aufweisen, die mit einer Führungseinrichtung zum Führen des Schlauchs versehen ist. Dabei kann die Führungseinrichtung als Schlitz in der Außenwand so vorgesehen sein, dass der Schlauch radial von außen und von unten in die Aufnahme einbringbar ist.

Dies ermöglicht vorteilhaft, dass rote Blutkörperchen, die durch den Schlauch gefördert wurden, sich an der Außenseite und Unterseite des Filters sammeln und keine Gefahr besteht, dass die roten Blutkörperchen in den Produktbeutel weitergefördert werden.

Es kann an der Oberseite der Trennwand eine Aussparung zur Führung des Schlauchs von der Aufnahme radial nach innen zu zweiten Aussparungen in der Oberseite der Abdeckung vorgesehen sein. Die zweiten Aussparungen sind dabei zur Aufnahme des Schlauchs und einer zweiten Schlauchklemme vorgesehen sind. Sie können insbesondere im Wesentlichen spiegelbildlich zu den ersten Aussparungen ausgeführt sein.

Die Zwischenwand kann an einer Oberkante eine Durchführung zur Durchführung des Schlauchs von dem radial inneren Bereich der Zwischenwand zu dem radial äußeren Bereich der Zwischenwand aufweisen. Dies dient u.a. der Positionierung des Schlauchs und der Sicherstellung, dass der Schlauch von radial außen und von unten zu dem Filter geführt ist.

Vorteilhaft kann in den zweiten Aussparungen ein zweiter lichtempfindlicher Sensor angeordnet sein und/oder in der Abdeckung den Betätigungseinrichtungen entsprechende zweite Betätigungseinrichtungen für eine in einer der zweiten Aussparungen aufnehmbare Schlauchklemme vorgesehen sein. Insbesondere der zweite Sensor ermöglicht es, die Ausbeute bei der Zellgewinnung zu optimieren, indem er nach einem durch den ersten Sensor ausgegebenen "Achtung"-Signal ausgehend von einer vorbestimmten Zusammensetzung des Produkts in dem Schlauch ein endgültiges Signal zum Beenden der Förderung verbunden mit einem Signal, die Schlauchklemmen zu schließen, ausgibt.

Der Einsatz kann außerdem mit einer radial außen liegenden Auffangwanne vorgesehen sein, die den Produktbereich und teilweise den Blutbeutelbereich umfassen kann. Vorteilhaft ist in der Auffangwanne eine Greifeinrichtung vorgesehen, die das Handhaben der Auffangwanne und des von dieser teilweise umfassten Einsatzes erleichtert. als Greifeinrichtung können beispielsweise Fingerlöcher oder Handgriffe vorgesehen sein.

Der voranstehend beschriebene Einsatz ist zur Verwendung in einer Zentrifuge zur Trennung von Blutbestandteilen vorgesehen. Die Zentrifuge weist eine Nabe und einen um diese drehbaren Rotor auf. In dem Rotor können vorteilhaft die Nabe herum angeordnete Aufnahmekästen, die auch als Systemboxen bezeichnet werden, angeordnet sein, die zur Aufnahme der Einsätze dienen. Allerdings ist auch eine Zentrifuge möglich, in der lediglich ein Einsatz aufgenommen ist. Jeder Einsatz ist durch ein Betätigen eines mit dem Aufnahmekasten verbundenen Verriegelungselements frei aus dem Aufnahmekasten bzw. der Zentrifuge entnehmbar. Die Aufnahmekästen können dabei lösbar mit dem Rotor verbunden sein.

Dies ermöglicht ein schnelles Austauschen der Einsätze mit den getrennten Blutbestandteilen durch neue Einsätze mit noch nicht getrennten Blutbestandteilen und ermöglicht dabei eine höhere Produktionsausbeute bei der Zellgewinnung und eine optimierte Geräteauslastung.

Das Verriegelungselement kann in einem nicht betätigten Zustand eine Verriegelungsposition einnehmen. Dadurch wird ein sofortiges Verriegeln erreicht, sobald ein Einsatz in den Aufnahmekasten des Rotors eingesetzt wird. Das Verriegelungselement kann außerdem in dem Bereich der Nabe oder des Aufnahmekastens vorgesehen sein.

Außerdem kann das Verriegelungselement bei einem dem Einsatz zugeordneten Stützteil vorgesehen sein. Entsprechend ist dann der Einsatz zwischen dem Stützteil und einer Wand des Aufnahmekastens aufgenommen und lediglich in eine Richtung nach oben bewegbar. Diese Bewegung ist allerdings nur durch die Betätigung des Verriegelungselements möglich. Dadurch ist eine sichere Positionierung des Einsatzes in dem Aufnahmekasten für den Zentrifugiervorgang möglich.

Zum Halten des Einsatzes kann das Verriegelungselement in dem nicht betätigten Zustand mit einer der Zwischenwand gegenüberliegenden Seitenfläche der Abdeckung in Eingriff sein. Auf dem Verriegelungselement ist dabei ein Vorsprung ausgebildet, der eine Bewegung des Einsatzes nach oben verhindert.

Das Stützteil kann außerdem eine Kontaktstelle zur Herstellung einer elektrisch leitenden Verbindung zwischen dem Aufnahmekasten und dem Einsatz aufweisen. Die Kontaktstelle kann dabei aus einer Vielzahl von elektrischen Kontaktpunkten bestehen.

Bei dem Stützteil kann ein Druckelement vorgesehen sein, das radial in den Bereich unter der Abdeckung beweglich ist. Dies dient dazu, die getrennten Bestandteile aus dem Blutbeutel heraus in den Schlauch zu drücken, durch den sie weiter zu dem Filter und in den Produktbeutel geleitet werden.

Ein erster Abschnitt einer Leitung des Blutbeutels kann vorteilhaft nach oben und radial nach innen geführt sein. Dies verhindert ein unerwünschtes Austreten von Flüssigkeiten in den Schlauch, bevor die Blutbestandteile voneinander getrennt sind.

Die Erfindung betrifft ebenfalls ein Verfahren zur Zellgewinnung. Das Verfahren weist die Folgenden Schritte auf: Einlegen eines Blutbeutels mit Vollblut in einen Blutbeutelbereich einer Einsatz, Schließen einer Abdeckung des Blutbeutelbereichs durch seitliches Verdrehen der Abdeckung und Positionieren eines Schlauchs des Blutbeutels, Verbinden des Schlauchs mit einem Filter in einem Produktbereich der Einsatz, Einsetzen der Einsatz in einen Rotor einer Zentrifuge unter Einrasten eines Verriegelungselements, Rotieren der Zentrifuge zur Auftrennung der Blutbestandteile in dem Blutbeutel, Ausüben von Druck mittels einem Druckelement auf den Blutbeutel, um dabei einen Blutbestandteil in den Schlauch und weiter in den Filter und einen hinter dem Filter angeordneten Produktbeutel zu drücken, Beenden der Druckausübung und der Rotation, Betätigung des Verriegelungselements und Ausbau der Einsatz, Öffnen der Abdeckung und Entnehmen der Beutel mit den getrennten Blutbestandteilen.

Das erfindungsgemäße Verfahren ermöglicht einerseits ein schnelleres Austauschen der Blutbeutel und Produktbeutel mit neuen Beuteln. Andererseits ist es durch die Möglichkeit, die Einsatzn mit einem Handgriff ein- und auszubauen möglich, den Zentrifugiervorgang und Trennvorgang nahezu ununterbrochen durchzuführen, da diese lediglich für das Austauschen der Einsatzn unterbrochen werden müssen.

Der erfindungsgemäße Einsatz und die Zentrifuge sind einerseits für die Gewinnung von Zellen und Plasma aus Vollblut geeignet, sind aber auch für die Gewinnung von Zellen aus dem nach einem bekannten Zentrifugiervorgang verbliebenen "buffy coat" vorgesehen.

Dazu wird das "buffy coat" aus mehreren Blutbeuteln gemeinsam mit einer Additivlösung in einem neuen Blutbeutel gesammelt und vermischt. Der neue Blutbeutel entspricht dabei dem Blutbeutel gemäß der Erfindung. Der neue Blutbeutel kann vorteilhaft mit einem Schlauch und/oder einem insbesondere zum Filtern von Leukozyten vorgesehenen Filter vorgesehen sein.

### Kurze Beschreibung der Abbildungen der Zeichnungen

Im Folgenden wird ein Ausführungsbeispiel der Erfindung anhand der Figuren beschrieben. Dabei zeigen:

Fig. 1 eine Draufsicht des erfindungsgemäßen Einsatzes,

Fig. 2 eine perspektivische Ansicht des Einsatzes,

Fig. 3 eine entlang einer Symmetrielinie geschnittene, perspektivische Ansicht des Einsatzes,

Fig. 4 eine die Ansicht der Fig. 4 ergänzende geschnittene, perspektivische Ansicht des Einsatzes,

Fig. 5 eine weitere perspektivische und geschnittene Ansicht des Einsatzes,

Fig. 6 eine Unterseite einer Abdeckung des Einsatzes,

Fig. 7 eine perspektivische Ansicht eines Aufnahmekastens,

Fig. 8a bis 8c schematische Schnittansichten des Einsatzes, aus denen die Zellgewinnung ersichtlich ist, und

Fig. 9 zeigt den Strom des Blutprodukts durch den Filter.
Weg(e) zur Ausführung der Erfindung

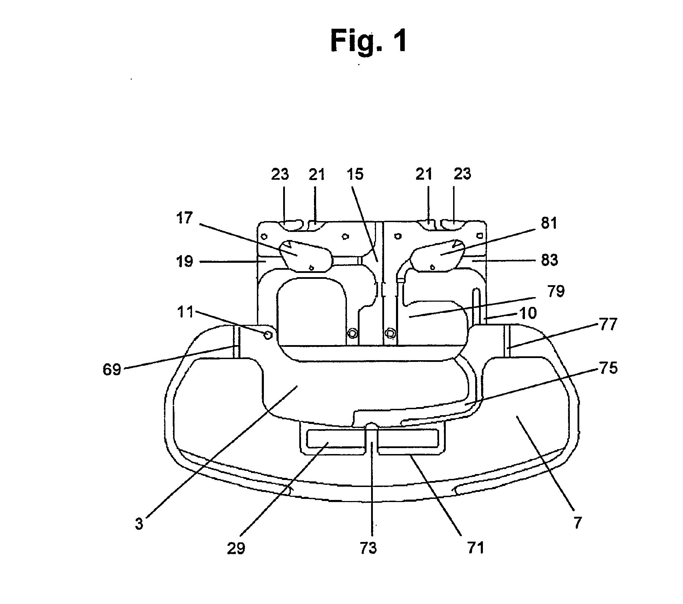

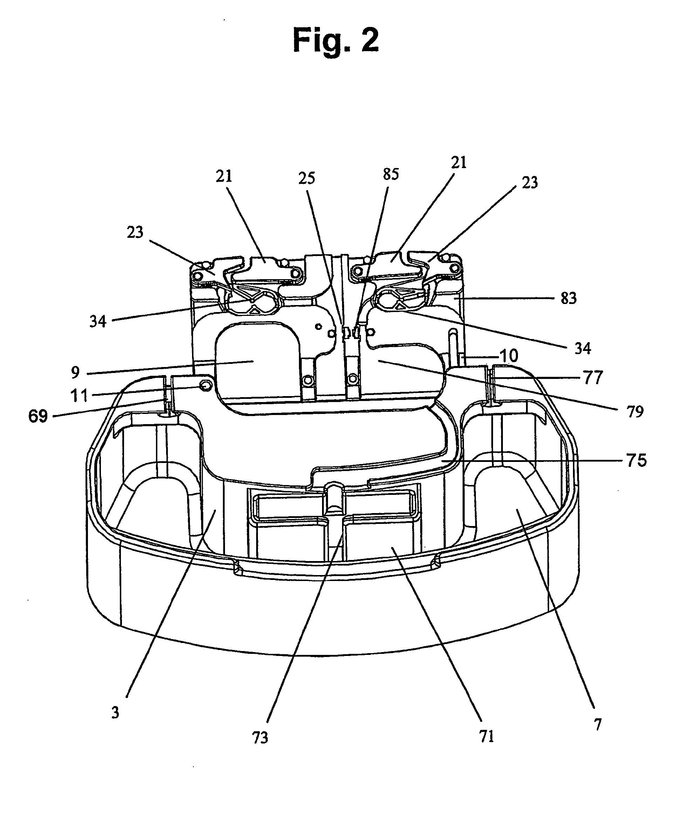

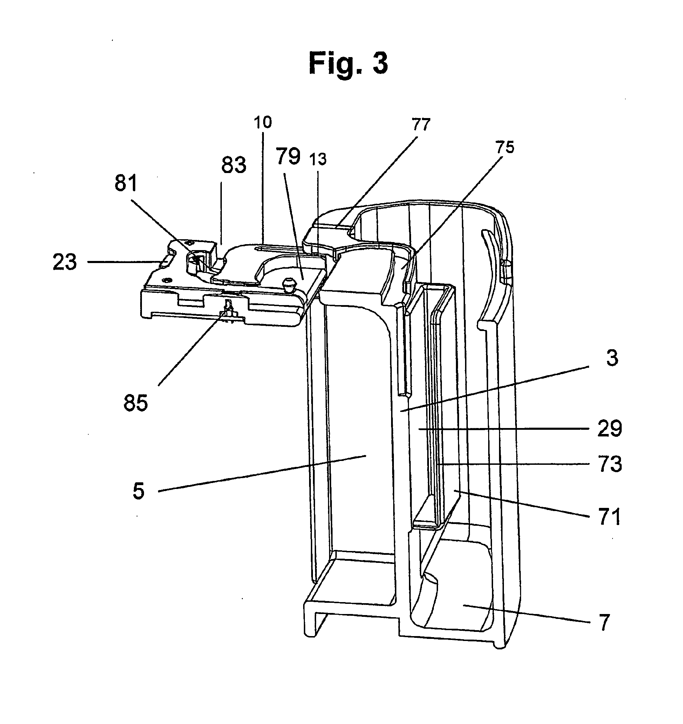

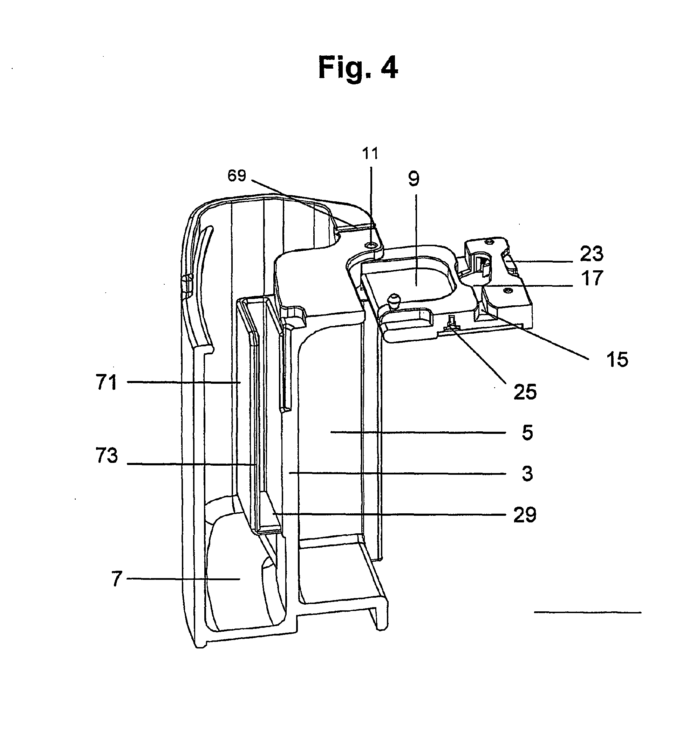

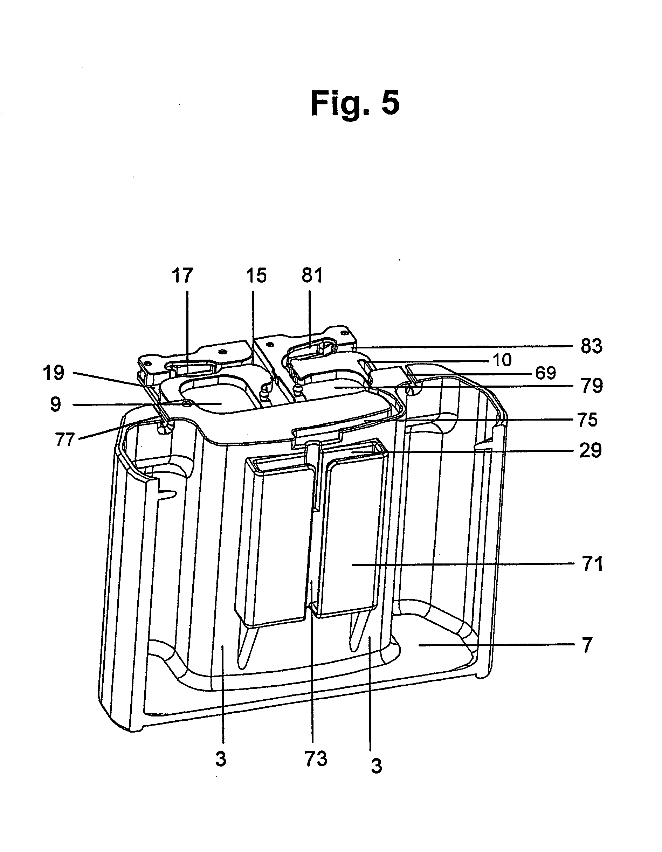

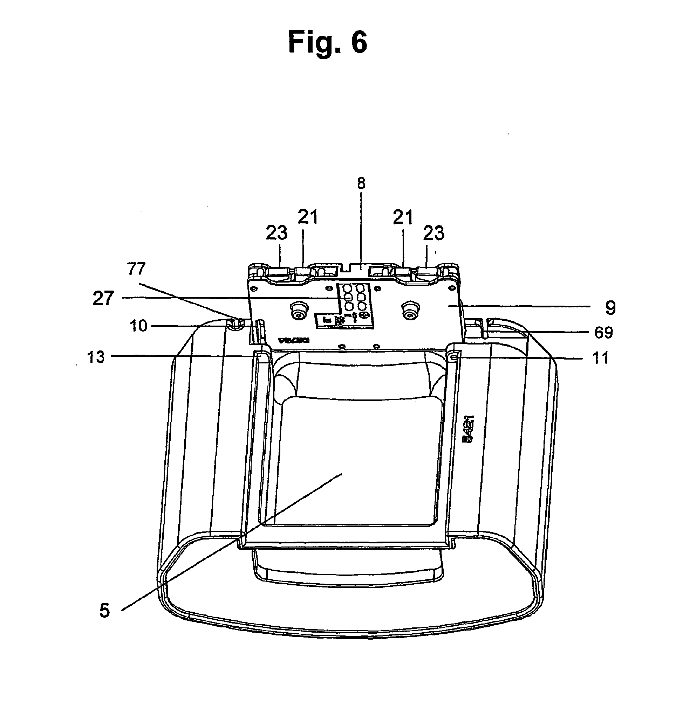

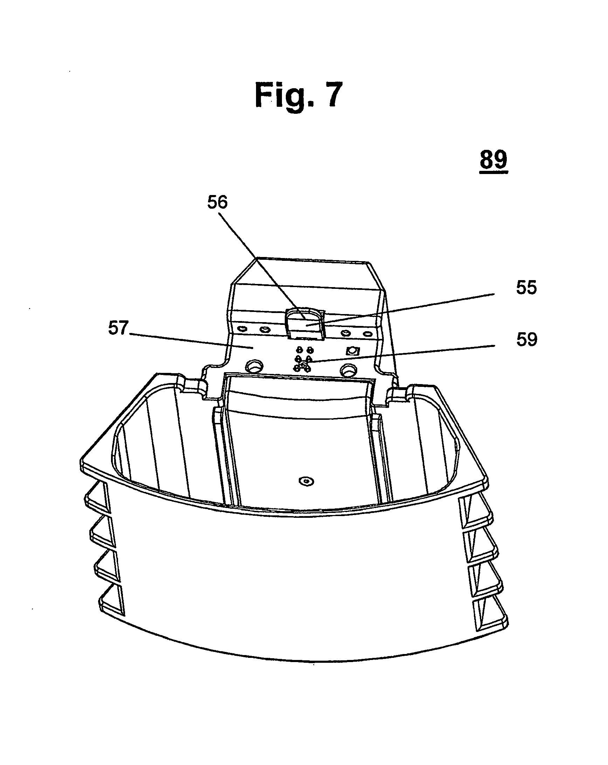

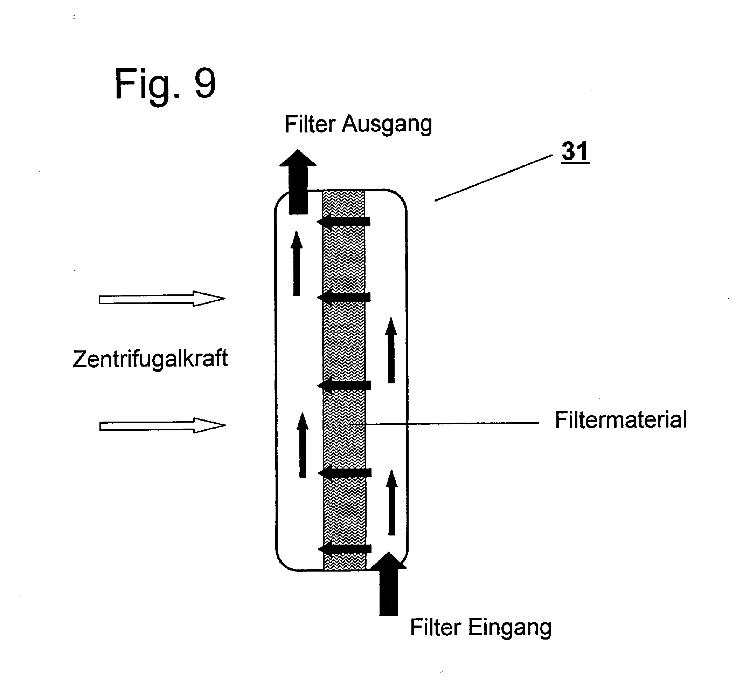

Ein Ausführungsbeispiel der Erfindung wird anhand der Figuren 1 bis 9 beschrieben.

Ein Einsatz 1 besteht im Wesentlichen aus einer Zwischenwand 3 und einer Abdeckung 9. Die Zwischenwand definiert einen Blutbeutelbereich 5 und einen Produktbeutelbereich 7. Ist der Einsatz 1 in eine Systembox 89 des Rotors einer Zentrifuge eingeschoben, liegt der Blutbeutelbereich 5 radial innerhalb von der Zwischenwand 3, während der Produktbeutelbereich 7 radial außerhalb von der Zwischenwand 3 liegt. Als Systembox 89 wird dabei ein Aufnahmekasten 89 gemäß der Erfindung bezeichnet.

Über dem Blutbeutelbereich 5 ist eine Abdeckung 9 vorgesehen. Diese ist im Wesentlichen rechteckig ausgebildet und in einem geschlossenen Zustand mit einer Längsseite mit der Zwischenwand 3 in Berührung. An einem Eckpunkt ist die Abdeckung drehbar in der Zwischenwand gelagert, während sie an einem zweiten Eckpunkt mittels einem Riegel 10 mit der Zwischenwand 3 in Eingriff gerät. Zum Öffnen der Abdeckung wird auf den Riegel 10 ein Druck ausgeübt, und danach die Abdeckung zur Seite gedreht. Dadurch ist der Blutbeutelbereich 5 frei zugänglich und kann mit einem Blutbeutel 35 befüllt werden.

Durch den einfachen Drehmechanismus kann ein Schlauch 36 und ein Blutbeutel 35 beim Schließen der Abdeckung 9 einfach und mit einem geringen Aufwand in einer vorgesehenen Lage gehalten und durch Schließen der Abdeckung 9 in dieser vorgesehenen Lage fixiert werden.

Nach dem Schließen der Abdeckung 9 ist es möglich, den Schlauch 36 in Aussparungen 15, 19 einzulegen, die an der Oberseite der Abdeckung 9 ausgebildet sind. Ein erster lichtempfindlicher Sensor 25 ist in der Aussparung 15 vorgesehen.

Eine mit dem Blutbeutel mitgelieferte, auf dem Schlauch vorhandene Schlauchklemme 34 in geschlossenem Zustand, beispielsweise eine von der Firma "Halkey Roberts" hergestellte, wird in einer ebenfalls an der Oberseite der Abdeckung 9 ausgebildeten Aussparung 17 aufgenommen.

Das von dem Blutbeutel 35 aus gesehene ferne Ende des Schlauchs 36 wird zu dem Produktbeutelbereich 7 geführt und ist dort mit einem Leukozytenfilter 31 verbunden, der in einer Aufnahme 29 aufgenommen ist. Dabei ist der Schlauch 36 radial von außen und von unten in den Leukozytenfilter 31 eingeführt. Das Einlegen von Filter 31 und Schlauch 36 wird durch einen Schlitz 73 in einer Außenwand 71 der Aufnahme 29 ermöglicht. Durch den Schlitz 73 kann der mit dem Filter 31 verbundene Schlauch 36 von oben nach unten verschoben werden, wenn der Filter in die Aufnahme eingebracht wird, sodass der Schlauch radial von außen und von unten zu dem Filter geführt ist.

Nach dem Filter 31 wird der Schlauch 36 über zweite in der Abdeckung 9 vorgesehene Aussparungen 75, 79, 81, 83, die im Wesentlichen spiegelbildlich zu den Aussparungen 15, 17, 19 angeordnet sind, zu dem Produktbeutel 33 geführt, der sich radial außerhalb von der Aufnahme 29 befindet.

In der Aussparung 81 ist eine zweite Schlauchklemme 34 vorgesehen. Ein zweiter lichtempfindlicher Sensor 85 befindet sich in der Aussparung 79.

Zum Betätigen der Klemmen 34 sind innerhalb der Abdeckung 9 jeweils zwei Stangen 21, 23 als Betätigungseinrichtung so durch die Abdeckung geführt, dass eines ihrer Enden geringfügig aus einer der Zwischenwand 3 gegenüberliegenden Seitenfläche 8 der Abdeckung 9 herausragt und das andere Ende sich in dem Bereich der die Klemme 34 aufnehmenden Vertiefung 17 befindet. Durch das Ausüben eines Drucks auf eines der aus der Seitenfläche 8 ragenden Enden kann somit die handelsübliche Klemme 34 geöffnet bzw. geschlossen werden. Gemäß der Ausführungsform sind die Schlauchklemmen 34 sowohl einzeln als auch pneumatisch betätigbar.

Nach der Bestückung des Einsatzes 1 kann der Einsatz 1 in die Systembox 89 des Rotors einer Zentrifuge eingesetzt werden. Dabei ruht die der Zwischenwand 3 gegenüberliegende Seitenfläche 8 der Abdeckung 9 an einem Stützteil 57 der Systembox 89, das in dem Bereich einer Nabe der Zentrifuge vorgesehen ist. Bei dem Stützteil 57 befindet sich außerdem ein stabförmiges Verriegelungselement 55, das an seiner radialen Außenseite einen Vorsprung 56 aufweist. Durch das Einsetzen des Einsatzes 1 gleitet die Seitenfläche 8 der Abdeckung 9 über den Vorsprung 56 und bewegt das Verriegelungselement 55 radial nach innen, bis die Seitenfläche 8 unter den Vorsprung 56 gerät, und das Verriegelungselement 55 in die ursprüngliche Lage zurückfedert, und somit eine Verschiebung des Einsatzes 1 nach oben verhindert. Der Einsatz 1 ist nun fest zwischen der Außenwand der Systembox 89 und dem Stützelement positioniert.

Gemäß dem Ausführungsbeispiel ist der Rotor der Zentrifuge für sechs Systemboxen 89 mit jeweils einem Einsatz 1 ausgelegt. Nach dem Einschieben aller Einsätze 1 wird die Zentrifuge gestartet. Durch die Fliehkraft kommt es zu der gewünschten Trennung der Blutbestandteile. Da sich das durch eine Additivlösung verdünnte "buffy coat" in dem Blutbeutel 35 befindet, werden dessen leichtere Bestandteile radial innen verbleiben, während schwerere Bestandteile d.h. die roten Blutkörperchen sich außen sammeln.

Um den gewünschten Blutbestandteil - gemäß der Ausführungsform sind dies die Blutplättchen - in hoher Qualität d.h. ohne Beimengung anderer Blutzellen aus dem Blutbeutel zu fördern, wird nach der Auftrennung der Bestandteile mittels einem bekannten Druckkissen 61 ein leichter Druck auf den Blutbeutel ausgeübt, sodass nach dem Öffnen der Klemmen 34 die plättchenreiche Lösung beginnt, in den nach oben und radial nach innen geführten Schlauch 36 aufzusteigen. Durch den Schlauch 36 wird die plättchenreiche Lösung in den Leukozytenfilter 31 geleitet, in den sie radial von außen und unten eintritt.

In dem Leukozytenfilter 31 werden die unerwünschten Leukozyten, das sind die weißen Blutkörperchen, entfernt. Aufgrund der erfindungsgemäßen Anordnung des Schlauchs 36 mit dem Filter 31 findet die Filtration entgegen der Zentrifugalkraft bzw. Fliehkraft statt. Dadurch werden schwerere Blutbestandteile wie ungewollt mitgeförderte rote Blutkörperchen in einer radial außen liegenden Filtereingangskammer abgefangen.

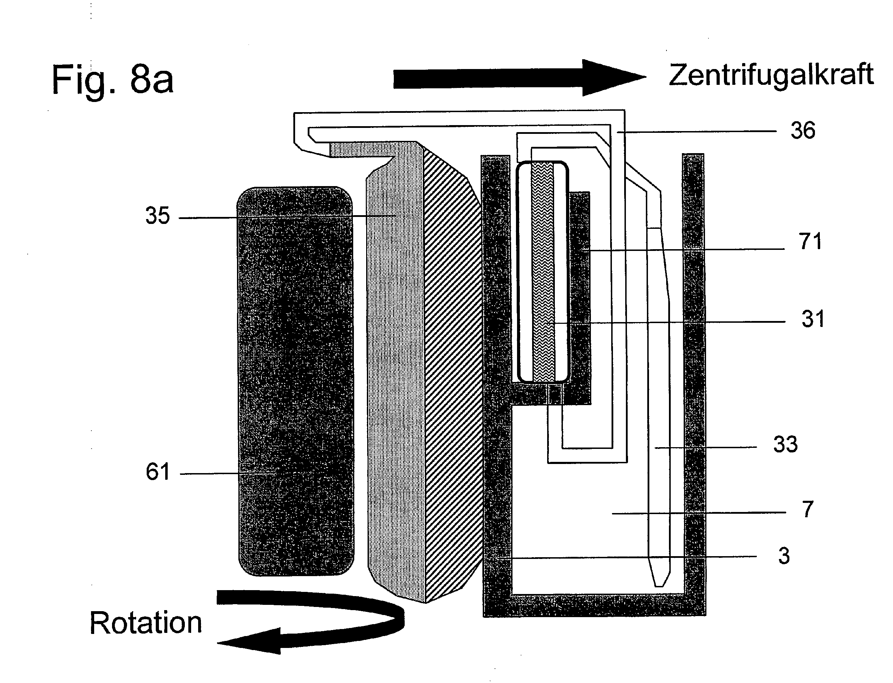

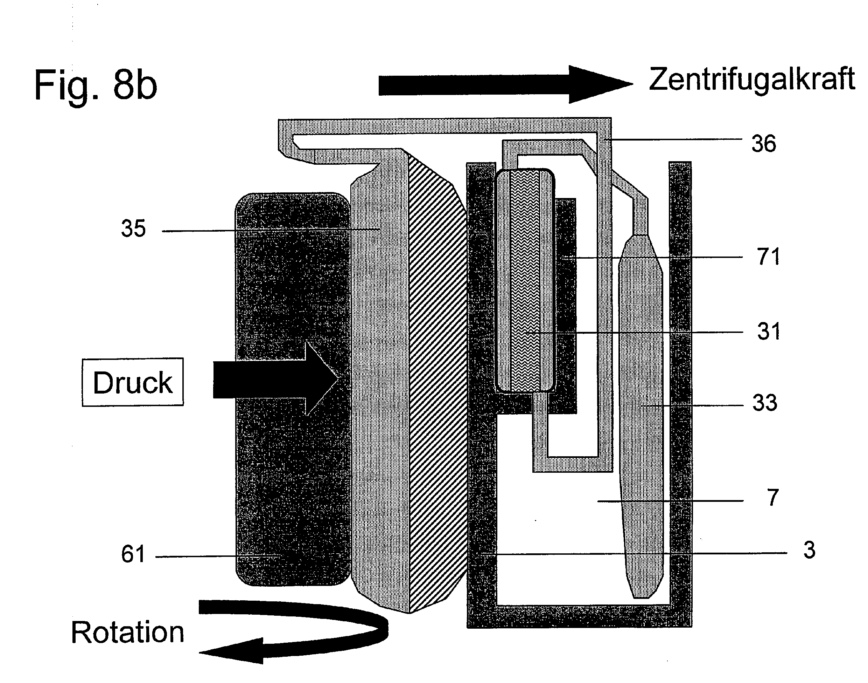

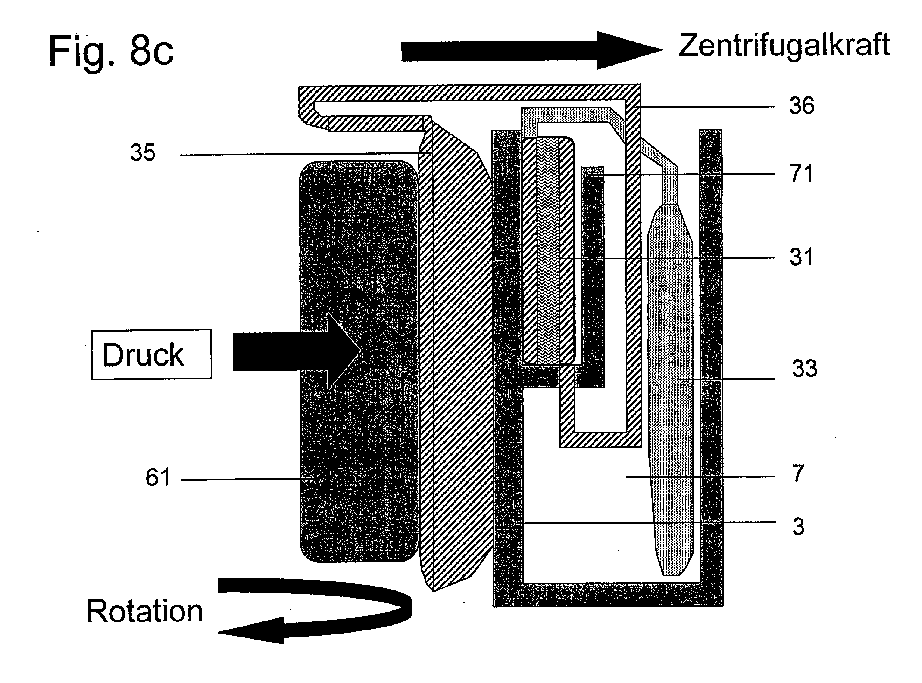

Nach dem Passieren des Leukozytenfilters 31 fließt die plättchenreiche Lösung schließlich weiter durch den Schlauch 36 in einen Produktbeutel 33, in dem sie gesammelt wird. Der Produktbeutel 33 ist vorzugsweise bereits als endgültiger Lagerbeutel für das Produkt ausgebildet. Der gesamte Vorgang ist in Fig. 8a bis 8c schematisch dargestellt.

Um eventuell vorhandene Luft in dem Filter zu entfernen, wird am Beginn der Produktüberleitung für eine bestimmte Volumenmenge die Fließgeschwindigkeit niedrig gehalten, und dadurch ermöglicht, dass sich der Filter zuverlässig und vollständig mit dem Blutprodukt füllt. Nach der Überleitung dieser bestimmten Volumenmenge wird mittels einer geeigneten Steuerung des Druckkissens, die Fördergeschwindigkeit für eine bestimmte zweite Volumenmenge erhöht. Während dieses zweite Volumen gefördert wird, besteht nur sehr geringe Gefahr, dass rote Blutkörperchen das Blutprodukt (hier das Thrombozytenkonzentrat) verunreinigen. Falls dies doch passiert, wird diese geringe Anzahl von roten Blutkörperchen, wegen der Führung des Schlauchs 36 von radial außen und unten in den Filter und der Wirkung der Zentrifugalkraft bzw. Fliehkraft, in dem unteren und äußeren Bereich des Filters gesammelt.

Nach dem Überleiten des zweiten Volumens wird der erste lichtempfindliche Sensor aktiviert und die Strömungsgeschwindigkeit des Blutprodukts in dem Schlauch 36 reduziert.

Erfasst der erste lichtempfindliche Sensor 25 einen vorbestimmten Anteil roter Blutkörperchen in der thrombozytenreichen Lösung, gibt er ein Signal ab, durch das die Strömungsgeschwindigkeit abermals reduziert wird. Außerdem wird der hinter dem Filter 31 angeordnete zweite lichtempfindliche Sensor 85 aktiviert.

In dieser Phase kann auch eine größere Anzahl roter Blutkörperchen in den Filter 31 eindringen und diesen sogar passieren, bis der zweite lichtempfindliche Sensor 85 einen vorbestimmten Anteil roter Blutkörperchen in dem Blutprodukt erfasst und ein Signal zum Beenden des Zellgewinnungsvorgangs ausgibt. Durch dieses Signal werden die Schlauchklemmen 34 durch das Betätigen der Stange 23 geschlossen, sodass die roten Blutkörperchen in dem Filter zuverlässig von dem Thrombozytenkonzentrat im Produktbeutel getrennt sind. Die Betätigung der Stange erfolgt über einen in der Systembox 89 vorgesehenen Stellmechanismus.

Alternativ zur Beendigung durch den zweiten lichtempfindlichen Sensor 85, kann der Zellgewinnungsvorgang auch nach dem Verstreichen eines bestimmten Zeitraums nach dem Aktivieren des zweiten lichtempfindlichen Sensors 85 beendet werden.

In der Ausführungsform sind insgesamt sechs Einsätze in der Zentrifuge vorgesehen. Durch die vorangehend beschriebene Steuerung des Zellgewinnungsvorgangs in einem Einsatz 1 mittels Druckkissen 61 dem Öffnen und Schließen der Schlauchklemmen 34, und der Prozeßkontrolle mittels der zwei lichtempfindlichen Sensoren 25, 85 ist es möglich, die Zellgewinnung in den Einsätzen der übrigen Systemboxen 89 fortzusetzen da die beschriebene Prozeßsteuerung individuell für jede Kombination aus Einsatz und Systembox stattfindet.

Zur Übertragung der Steuer- und anderer elektrischer Signale ist auf dem Stützteil 57 der Systembox 89 eine elektrische Kontaktstelle in Form von einzelnen Kontaktpunkten 59 vorgesehen. An der Unterseite der Abdeckung 9 befinden sich den Kontaktpunkten 59 zugeordnete Kontaktflächen 27, die bei dem Einschieben des Einsatzes 1 in die Systembox mit den Kontaktpunkten 59 in Berührung geraten. Die Kontaktpunkte 59 sind zu diesem Zweck federnd gelagert.

Zur leichteren Handhabung einerseits und für den Fall, bei dem Blutbestandteile aufgrund von einer Beschädigung der Beutel 33, 35, des Schlauchs 36 oder des Filters 31 austreten, ist der Einsatz 1 von einer radial inneren Richtung in eine Auffangwanne eingeführt. In dem Fall einer Beschädigung wird der ausgetretene Blutbestandteil zum Großteil in der Auffangwanne gesammelt, sodass es nur zu geringfügigen Verschmutzungen der Systembox 89 oder des Rotors selbst kommt. Die Systembox 89 kann in einem derartigen Fall einfach aus dem Rotor ausgebaut werden.

Nach der Beendigung der Zellgewinnung wird jeder der Einsätze 1 entnommen, indem auf das Verriegelungselement 55 ein leichter Druck ausgeübt wird, um dieses radial nach innen zu bewegen.

Gleichzeitig werden die Einsätze 1 an Fingerlöchern der Auffangwanne erfasst und nach oben aus der Systembox 89 der Zentrifuge herausgehoben, um sofort durch neue, frisch bestückte Einsätze 1 ersetzt zu werden. Während der folgenden Zellgewinnung können die Blutbeutel 35 und Produktbeutel 33 aus den ausgetauschten Einsätzen 1 entnommen werden und diese wieder neu bestückt werden.

Die Erfindung betrifft einen Einsatz (1) zur Aufnahme von Blutbeuteln (35) der für die Trennung von Blutbestandteilen zum Einsatz in einer Zentrifuge vorgesehen ist. Der Einsatz (1) hat eine Zwischenwand (3), die einen radial innen liegenden Blutbeutelbereich (5) von einem radial außen liegenden Produktbereich (7) abteilt, und

einer in einer Einbauposition über dem Blutbeutelbereich (5) liegende Abdeckung (9). Die Abdeckung (9) ist an einem ersten Punkt (11) drehbar und an einem zweiten Punkt (13) lösbar mit der Zwischenwand (3) verbunden, sodass der Blutbeutelbereich (5) durch ein seitliches Wegdrehen der Abdeckung (9) frei zugänglich ist. Der Einsatz ist in dem Rotor einer Zentrifuge einsetzbar.

## Patentansprüche

1. Einsatz (1) zur Aufnahme von Blutbeuteln (35) zum Einsatz in einer Zentrifuge zur Trennung von Blutbestandteilen, mit:
einer Zwischenwand (3), die einen radial innen liegenden Blutbeutelbereich (5) von einem radial außen liegenden Produktbereich (7) abteilt, und
einer in einer Einbauposition über dem Blutbeutelbereich (5) liegenden Abdeckung (9),
**dadurch gekennzeichnet, dass** die Abdeckung (9) an einem ersten Punkt (11) drehbar und an einem zweiten Punkt (13) lösbar mit der Zwischenwand (3) verbunden ist, sodass der Blutbeutelbereich (5) durch ein seitliches Wegdrehen der Abdeckung (9) frei zugänglich ist.

2. Einsatz (1) nach Anspruch 1, wobei in einem oberen Bereich der Abdeckung (9) eine Aussparung (15, 17,19) zur Aufnahme eines von dem Blutbeutel (35) wegführenden Schlauchs (36) und/oder einer Schlauchklemme (34) ausgebildet ist.

3. Einsatz (1) nach einem der Ansprüche 1 oder 2, wobei in der Abdeckung (9) eine Betätigungseinrichtung (21, 23) zum Betätigen einer auf einem Schlauch (36) des Blutbeutels (35) vorhandenen Schlauchklemme (34) vorgesehen ist, wobei vorzugsweise die Betätigungseinrichtung (21,23) an einer bei geschlossener Abdeckung (9) der Zwischenwand (3)gegenüberliegenden Seitenfläche (8) aus der Abdeckung (9) vorsteht.

4. Einsatz (1) nach einem der Ansprüche 2 oder 3, wobei in der Aussparung (15, 17, 19) ein lichtempfindlicher Sensor (25) vorgesehen ist.

5. Einsatz (1) nach einem der Ansprüche 1 bis 4, wobei an einer Unterseite der Abdeckung (9) eine elektrische Verbindungseinrichtung (27) zur elektrischen Verbindung mit der Zentrifuge vorgesehen ist.

6. Einsatz (1) nach einem der Ansprüche 1 bis 5, wobei radial außerhalb von dem Blutbeutelbereich (5) eine Aufnahme (29) für einen Filter (31) vorgesehen ist,
wobei vorzugsweise die Aufnahme (29) eine radial außerhalb der Zwischenwand (3) liegende Außenwand (71) aufweist, die mit einer Führungseinrichtung (73) zum Führen des Schlauchs (36) versehen ist,
wobei besonders vorteilhaft die Führungseinrichtung (73) als Schlitz (73) in der Außenwand so vorgesehen ist, dass der Schlauch (36) radial von außen und von unten in die Aufnahme (29) einbringbar ist.

7. Einsatz (1) nach Anspruch 6, wobei an der Oberseite der Trennwand (3) eine Aussparung (75) zur Führung des Schlauchs (36) von der Aufnahme (29) radial nach innen zu zweiten Aussparungen (79, 81, 83) in der Oberseite der Abdeckung (9) vorgesehen ist, und die zweiten Aussparungen (79, 81, 83) zur Aufnahme des Schlauchs (36) und einer zweiten Schlauchklemme (34) vorgesehen sind,
wobei vorzugsweise die zweiten Aussparungen (79, 81, 83) im Wesentlichen spiegelbildlich zu den ersten Aussparungen (15, 17, 19) vorgesehen sind,
wobei besonders vorteilhaft die Zwischenwand (3) an einer Oberkante eine Durchführung (69, 77) zur Durchführung des Schlauchs (36) von dem radial inneren Bereich der Zwischenwand (3) zu dem radial äußeren Bereich der Zwischenwand (3) aufweist.

8. Einsatz nach Anspruch 7, wobei in den zweiten Aussparungen (79, 81, 83) ein zweiter lichtempfindlicher Sensor (85) angeordnet ist und/oder in der Abdeckung den Betätigungseinrichtungen (21, 23) entsprechende zweite Betätigungseinrichtungen (21,23) für eine in einer der zweiten Aussparungen (79, 81, 83) aufnehmbare Schlauchklemme (34) vorgesehen sind.

9. Einsatz nach einem der Ansprüche 1 bis 8, wobei eine radial außen liegende Auffangwanne (87) vorgesehen ist, die den Produktbereich (7) und teilweise den Blutbeutelbereich (5) umfasst,
wobei vorzugsweise die Auffangwanne (87) mit einer Greifeinrichtung (88) zur Handhabung derselben und des Einsatzes versehen ist.

10. Zentrifuge zur Trennung von Blutbestandteilen mit:
einem um eine Nabe (51) drehbaren Rotor, mindestens einem in dem Rotor aufgenommenen und teilweise um die Nabe herum angeordneten Einsatz (1) nach einem der Ansprüche 1 bis 9,
zur Aufnahme von Blutbeuteln (35),
**gekennzeichnet durch**
ein mit dem Rotor verbundenes Verriegelungselement (55),
das dem Einsatz (1) zugeordnet ist, und bei dessen Betätigung der Einsatz (1) frei aus dem Rotor entnehmbar ist.

11. Zentrifuge nach Anspruch 10, wobei in dem Rotor mindestens ein Aufnahmekasten (89) zur Aufnahme eines Einsatzes (1) vorgesehen ist,
wobei vorzugsweise der Aufnahmekasten (89) lösbar mit dem Rotor verbunden ist und das Verriegelungselement (55) an dem Aufnahmekasten vorgesehen ist.

12. Zentrifuge nach einem der Ansprüche 10 bis 11, wobei der Einsatz (1) in einer radial außen liegenden Auffangwanne (87) aufgenommen ist und zusammen mit dieser in den Rotor einsetz- und entnehmbar sind.

13. Zentrifuge nach einem der Ansprüche 10 bis 12, wobei das Verriegelungselement (55) bei der Nabe (51) des Rotors angeordnet ist,
wobei vorzugsweise das Verriegelungselement (55) bei einem dem Einsatz (1) zugeordneten Stützteil (57) vorgesehen ist, wobei besonders vorteilhaft das Verriegelungselement (55) in dem nicht betätigten Zustand mit einer der Zwischenwand (3) gegenüberliegenden Seitenfläche (8) der Abdeckung (9) in Eingriff ist und ein Vorsprung (56) auf dem Verriegelungselement (55) eine Bewegung des Einsatzes (1) nach oben verhindert.

14. Zentrifuge nach Anspruch 13, wobei das Stützteil (57) und/oder der Aufnahmekasten (89) eine Kontaktstelle (59) zur Herstellung einer elektrisch leitenden Verbindung zwischen dem Rotor und dem Einsatz (1) aufweist,
wobei vorzugsweise die Kontaktstelle aus einer Vielzahl elektrischer Kontaktpunkte (59) besteht.

15. Zentrifuge nach einem der Ansprüche 10 bis 14, wobei in dem Bereich der Nabe (51) des Rotors ein Stellmechanismus zur Betätigung von im Einsatz vorgesehenen Betätigungseinrichtungen (21, 23) vorgesehen ist,
wobei vorzugsweise die Betätigung durch den Stellmechanismus pneumatisch oder elektrisch oder hydraulisch erfolgt.

16. Zentrifuge nach einem der Ansprüche 10 bis 15, wobei in dem Bereich der Nabe (51) ein radial nach außen verschiebbares Druckelement (61) zum Ausüben eines Drucks auf einen Blutbeutel (35) vorgesehen ist.

17. Verfahren zur Zellgewinnung aus Blut oder einem Blutbestandteil mit den Schritten:
Einlegen eines Blutbeutels (35) mit Vollblut oder
Blutbestandteilen in einen Blutbeutelbereich (5) eines Einsatzes (1),
Schließen einer Abdeckung (9) des Blutbeutelbereichs (5) durch seitliches Verdrehen der Abdeckung (9) und
Positionieren eines Schlauchs (36) des Blutbeutels mit einem Filter (31) in einem Produktbereich (7) des Einsatzes (1),
Einsetzen des Einsatzes (1) in einen Rotor einer Zentrifuge unter Einrasten eines Verriegelungselements (55),
Rotieren der Zentrifuge zur Auftrennung der Blutbestandteile in dem Blutbeutel (35),
Ausüben von Druck mittels einem Druckelement (61) auf den Blutbeutel, um dabei einen Blutbestandteil in den Schlauch (36) und weiter in den Filter (31) und einen hinter dem Filter angeordneten Produktbeutel (33) zu drücken,
Beenden der Druckausübung und der Rotation,
Betätigung des Verriegelungselements (55) und Ausbau des Einsatzes (1),
Öffnen der Abdeckung (9) und Entnehmen der Beutel (33, 35) mit den getrennten Blutbestandteilen.

## Claims

1. A cartridge (1) for accommodating blood bags (35) to be used in a centrifuge for the separation of blood components, comprising:
a partition wall (3) which separates a blood bag section (5) which is positioned radially inside from a product section (7) which is positioned radially outside, and
a cover (9) which is disposed in a mounting position above the blood bag section (5),
**characterized in that**
the cover (9) is connected to the partition wall (3) pivotally in a first point (11) and detachably in a second point (13), so that the blood bag section (5) is freely accessible by means of laterally pivoting the cover (9) out of the way.

2. A cartridge (1) according to claim 1, wherein a recess (15, 17, 19) for holding a tube (36) leading away from the blood bag (35) and/or a tube clamp (34) is formed in an upper section of the cover (9).

3. A cartridge (1) according to claim 1 or 2, wherein an operating device (21, 23) is provided in the cover (9) for operating a tube clamp (34) disposed on a tube (36) of the blood bag (35), wherein preferably the operating device (21, 23) protrudes from the cover (9) at a side surface (8) located opposite the partition wall (3) when the cover (9) is closed.

4. A cartridge (1) according to one of claims 2 or 3, wherein a photo sensor (25) is provided in the recess (15, 17, 19).

5. A cartridge (1) according to one of claims 1 to 4, wherein an electric connection means (27) for the electrical connection with the centrifuge is provided at a bottom surface of the cover (9).

6. A cartridge (1) according to one of claims 1 to 5, wherein a fixture (29) for a filter (31) is provided radially outside of the blood bag section (5) wherein preferably the fixture (29) comprises an outer wall (71) positioned radially outside the partition wall (3) and having a guiding means (73) for guiding the tube (36), wherein especially advantageous the guiding means (73) is provided as a slot (73) in the outside wall in such way that the tube (36) can be inserted radially from the outside and from below into the fixture (29).

7. A cartridge (1) according to one of claim 6, wherein, at the top surface of the partition wall (3), a recess (75) is provided for guiding the tube (36) from the fixture (29) radially inward to second recesses (79, 81, 83) in the top surface of the cover (9), and the second recesses (79, 81, 83) are provided for holding the tube (36) and a second tube clamp (34), wherein preferably the second recesses (79, 81, 83) are essentially positioned in a mirror-image manner relative to the first recesses (15, 17, 19), wherein especially advantageous the partition wall (3) comprises a passage (69, 77) at an upper edge for feeding the tube (36) from the radially inside section of the partition wall (3) to the radially outside section of the partition wall (3).

8. A cartridge according to claim 7, wherein a second photo sensor (85) is disposed in the second recesses (79, 81, 83), and/or second operating device (21, 23) corresponding to the operating device (21, 23) for a tube clamp (34) to be held in one of the second recesses (79, 81, 83) are provided in the cover.

9. A cartridge according to one of claims 1 to 8, wherein a collecting tank (87) positioned radially outside is provided and embraces the product section (7) and parts of the blood bag section (5), wherein preferably the collecting tank (87) is provided with a handling means (88) for handling the tank and the cartridge.

10. A centrifuge for the separation of blood components, comprising:
a rotor revolving about a hub (51),
at least one cartridge (1) according to one of claims 1 to 9, held in the rotor and partly arranged around the hub for accommodating blood bags (35),
**characterized by**
a locking element (55) connected to the rotor and
assigned to the cartridge (1), upon the operation of which the cartridge (1) can be removed freely from the rotor.

11. A centrifuge according to claim 10, wherein at least one accommodating box (89) for accommodating a cartridge (1) is provided in the rotor, wherein preferably the accommodating box (89) is detachably connected to the rotor and the locking element (55) is provided at the accommodating box.

12. A centrifuge according to one of claims 10 to 11, wherein the cartridge (1) is accommodated in a collecting tank (87) positioned radially outside, and can be inserted into and removed from the rotor jointly with the same.

13. A centrifuge according to one of claims 10 to 12, wherein the locking element (55) is positioned at the hub (51) of the rotor, wherein preferably the locking element (55) is provided at a support (57) assigned to the cartridge (1), wherein especially advantageous the locking element (55), in its non-operated state, is engaged with a side surface (8) of the cover (9), located opposite the partition wall (3), and a projection (56) on the locking element (55) prevents an upward movement of the cartridge (1).

14. A centrifuge according to claim 13, wherein the support (57) and/or the accommodating box (89) is provided with a contact pad (59) for establishing an electrically conductive connection between the rotor and the cartridge (1), wherein preferably the contact pad consists of a plurality of electric contact points (59).

15. A centrifuge according to one of claims 10 to 14, wherein, in the area of the hub (51) of the rotor, an actuating mechanism is provided for operating the operating device (21, 23) provided in the cartridge, wherein preferably the operation of the actuating mechanism is effected pneumatically or electrically or hydraulically.

16. A centrifuge according to one of claims 10 to 15, wherein, in the area of the hub (51), a pressing element (61) which can be displaced radially outwards is provided for applying pressure onto a blood bag (35).

17. A method for cell isolation from blood or from a blood component, comprising the steps of:
inserting a blood bag (35) containing whole blood or blood components into a blood bag section (5) of a cartridge (1),
closing a cover (9) of the blood bag section (5) by pivoting the cover (9) to the side, and positioning a tube (36) of the blood bag including a filter (31) in a product section (7) of the cartridge (1),
inserting the cartridge (1) into a rotor of a centrifuge by latching a locking element (55),
spinning the centrifuge in order to separate the blood components in the blood bag (35),
applying pressure onto the blood bag by means of a pressure element (61) in order to press a blood component into the tube (36) and further into the filter (31) and into a product bag (33) positioned behind the filter,
terminating the pressing and the spinning,
operating the locking element (55) and removal of the cartridge (1),
opening the cover (9) and removing the bags (33, 35) containing the separated blood components.

## Revendications

1. Insert (1) pour la réception de poches de sang (35) destiné à être inséré dans une centrifugeuse pour la séparation des composants du sang, comprenant
une paroi intermédiaire (3) qui divise une zone de poche de sang (5) se trouvant radialement à l'intérieur d'une zone de produit se trouvant radialement à l'extérieur, et
un recouvrement (9) se trouvant dans une position de montage au-dessus de la zone de poche de sang (5),
**caractérisé en ce que**
le recouvrement (9) est relié de manière rotative sur un premier point (11) et de manière détachable sur un second point (13) à la paroi intermédiaire (3) de sorte que la zone de poche de sang (5) soit librement accessible en enlevant le recouvrement (9) par rotation latérale.

2. Insert (1) selon la revendication 1, un évidement (15, 17, 19) pour la réception d'un tuyau (36) s'éloignant de la poche de sang (35) et/ou d'une pince pour tuyau (34) étant réalisé dans une zone supérieure du recouvrement (9).

3. Insert (1) selon l'une quelconque des revendications 1 ou 2, un dispositif d'actionnement (21, 23) pour l'actionnement d'une pince pour tuyau (34) présente sur un tuyau (36) de la poche de sang (35) étant prévu dans le recouvrement (9), le dispositif d'actionnement (21, 23) faisant saillie de préférence du recouvrement (9) sur une face latérale (8) opposée à la paroi intermédiaire (3) en cas de recouvrement (9) fermé.

4. Insert (1) selon l'une quelconque des revendications 2 ou 3, un capteur (25) photosensible étant prévu dans l'évidement (15, 17, 19).

5. Insert (1) selon l'une quelconque des revendications 1 à 4, un dispositif de liaison (27) électrique pour la liaison électrique avec la centrifugeuse étant prévu sur un côté inférieur du recouvrement (9).

6. Insert (1) selon l'une quelconque des revendications 1 à 5, un logement (29) pour un filtre (31) étant prévu radialement à l'extérieur de la zone de poche de sang (5),
le logement (29) présentant de préférence une paroi extérieure (71) se trouvant radialement à l'extérieur de la paroi intermédiaire (3), qui est pourvue d'un dispositif de guidage (73) pour le guidage du tuyau (36),
le dispositif de guidage (73) étant prévu de manière particulièrement avantageuse comme une fente (73) dans la paroi extérieure de sorte que le tuyau (36) puisse être introduit radialement de l'extérieur et par le bas dans le logement (29).

7. Insert (1) selon la revendication 6, un évidement (75) pour le guidage du tuyau (36) du logement (29) radialement vers l'intérieur jusqu'à des seconds évidements (79, 81, 83) dans le côté supérieur du recouvrement (9) étant prévu sur le côté supérieur de la paroi de séparation (3), et les seconds évidements (79, 81, 83) étant prévus pour le logement du tuyau (36) et d'une seconde pince pour tuyau (34),
les seconds évidements (79, 81, 83) étant prévus de préférence de manière sensiblement inversée par rapport aux premiers évidements (15, 17, 19),
la paroi intermédiaire (3) présentant de manière particulièrement avantageuse sur une arête supérieure un passage (69, 77) pour le passage du tuyau (36) de la zone radialement intérieure de la paroi intermédiaire (3) à la zone radialement extérieure de la paroi intermédiaire (3).

8. Insert selon la revendication 7, un second capteur (85) photosensible étant disposé dans les seconds évidements (79, 81, 83) et/ou des seconds dispositifs d'actionnement (21, 23), correspondant aux dispositifs d'actionnement (21, 23), pour une pince pour tuyau (34) pouvant être reçue dans l'un des seconds évidements (79 81, 83) étant prévus dans le recouvrement.

9. Insert selon l'une quelconque des revendications 1 à 8, une cuve collectrice (87) se trouvant radialement à l'extérieur étant prévue, laquelle comporte la zone de produit (7) et en partie la zone de poche de sang (5),
la cuve collectrice (87) étant pourvue de préférence d'un dispositif de préhension (88) pour sa manipulation et celle de l'insert.

10. Centrifugeuse pour la séparation de composants du sang comprenant :
un rotor pouvant tourner autour d'un moyeu (51), au moins un insert (1) reçu dans le rotor et disposé en partie autour du moyeu selon l'une quelconque des revendications 1 à 9 pour la réception de poches de sang (35),
**caractérisée par** un élément de verrouillage (55) relié au rotor et associé à l'insert (1), et lors de l'actionnement duquel, l'insert (1) peut être retiré librement du rotor.

11. Centrifugeuse selon la revendication 10, au moins un caisson récepteur (89) pour la réception d'un insert (1) étant prévu dans le rotor,
le caisson récepteur (89) étant relié de préférence de manière détachable au rotor et l'élément de verrouillage (55) étant prévu sur le caisson récepteur.

12. Centrifugeuse selon l'une quelconque des revendications 10 à 11, l'insert (1) étant reçu dans une cuve collectrice (87) se trouvant radialement à l'extérieur et pouvant être inséré et retiré conjointement avec celle-ci du rotor.

13. Centrifugeuse selon l'une quelconque des revendications 10 à 12, l'élément de verrouillage (55) étant disposé près du moyeu (51) du rotor,
l'élément de verrouillage (55) étant prévu de préférence près d'une partie d'appui (57) associée à l'insert (1),
l'élément de verrouillage (55) étant en engagement de manière particulièrement avantageuse à l'état non actionné avec une face latérale (8) du recouvrement (9) opposée à la paroi intermédiaire (3) et une saillie (56) sur l'élément de verrouillage (55) empêchant un mouvement de l'insert (1) vers le haut.

14. Centrifugeuse selon la revendication 13, la partie d'appui (57) et/ou le caisson récepteur (89) présentant un point de contact (59) pour l'établissement d'une liaison électroconductrice entre le rotor et l'insert (1), le point de contact se composant de préférence d'une pluralité de points de contact électriques (59).

15. Centrifugeuse selon l'une quelconque des revendications 10 à 14, un mécanisme de commande pour l'actionnement de dispositifs d'actionnement (21, 23) prévus dans l'insert étant prévu dans la zone du moyeu (51) du rotor, l'actionnement étant de préférence effectué par le mécanisme de commande par voie pneumatique, électrique ou hydraulique.

16. Centrifugeuse selon l'une quelconque des revendications 10 à 15, un élément de compression (61) mobile radialement vers l'extérieur pour l'exercice d'une pression sur une poche de sang (35) étant prévu dans la zone du moyeu (51).

17. Procédé d'extraction de cellules du sang ou d'un composant du sang comportant les étapes suivantes :
insertion d'une poche de sang (35) ne contenant que du sang ou des composants du sang dans une zone de poche de sang (5) d'un insert (1),
fermeture d'un recouvrement (9) de la zone de poche de sang (5) par rotation latérale du recouvrement (9) et
positionnement d'un tuyau (36) de la poche de sang avec un filtre (31) dans une zone de produit (7) de l'insert (1),
insertion de l'insert (1) dans un rotor d'une centrifugeuse en encliquetant un élément de verrouillage (55),
rotation de la centrifugeuse pour la séparation des composants du sang dans la poche de sang (35),
exercice de la pression au moyen d'un élément de compression (61) sur la poche de sang afin de pousser un composant du sang dans le tuyau (36) et plus encore dans le filtre (31) et dans une poche de produit (33) disposée derrière le filtre,
fin de l'exercice de la pression et de la rotation,
actionnement de l'élément de verrouillage (55) et démontage de l'insert (1),
ouverture du recouvrement (9) et retrait des poches (33, 35) contenant les composants du sang séparés.
